# EUROPEAN PATENT SPECIFICATION

(11) **EP 4 009 986 B1**
(45) Date of publication and mention of the grant of the patent: **22.04.2026**
(21) Application number: 20850476.1
(22) Date of filing: 10.07.2020
(51) Int. Cl.: A61K 47/10, A61K 9/00, A61K 47/02, A61K 31/77, A61K 33/00, A61K 33/14, A61P 1/10

(54) **A SACHET FORMULATION COMPRISING POLYETHYLENE GLYCOL**
BEUTELFORMULIERUNG MIT POLYETHYLENGLYKOL
FORMULATION EN SACHET COMPRENANT DU POLYÉTHYLÈNE GLYCOL

(30) Priority: 07.08.2019 TR 201912004
(43) Date of publication of application: 15.06.2022
(73) Proprietor: Sanovel Ilac Sanayi ve Ticaret A.S., Sisli - Istanbul (TR)
(72) Inventor: PEHLIVAN AKALIN, Nur, 34460 Sar yer/Istanbul (TR); OZDEN, Aydan, 34460 Sar yer/Istanbul (TR); GULER, Tolga, 34460 Sar yer/Istanbul (TR)
(74) Representative: Bakirci, Utkan Bahri
(86) International application number: PCT/TR2020/050616
(87) International publication number: WO 2021/025647

(56) References cited:
- AU-A1- 2015 275 229
- CN-A- 101 036 668
- CN-B- 102 772 427
- GB-A- 2 471 954
- ANONYMOUS: "GaviLyte-H", RXLIST.COM, 27 December 2016 (2016-12-27), XP055771296, Retrieved from the Internet <URL:https://www.rxlist.com/gavilyte-h-drug.htm> [retrieved on 20210202]

## Description

### Field of the invention

The present invention relates to a sachet formulation comprising polyethylene glycol for the treatment of constipation, colon evacuation or colon cleansing. The invention further relates to a process of the formulation for effective and safe use.

### Background of the invention

Constipation is a widespread condition which generally gives rise to discomfort and unrest. The physical presence of faeces retained in the colon and/or the rectum gives rise to a feeling of malaise and headaches. In extreme cases of prolonged constipation dyschezia may result from the presence of scybala or faecaliths in the rectum. Numerous treatments of constipation have been developed, including dietary manipulation (e.g. increasing the fibre content of the diet and removing foods considered to be constipation causing), laxatives and enemas. Laxatives are agents that promote and assist defecation.

One method of colon cleansing is orthostatic intestinal lavage, in which a large volume of an electrolyte solution is ingested, either by drinking or by infusion through a nasogastric tube. Such lavage solutions are also known as bowel or gut lavage solutions. Consumption of the solution results in volume-induced diarrhoea and thus cleansing of the colon. Most commonly used lavage solutions include polyethylene glycol (PEG).

A number of osmotic laxative treatments currently in use comprise polyethylene glycol. Such compositions may also include electrolytes. One laxative that comprises PEG and electrolytes that is currently on the market is Movicol^{®}. It is supplied in sachets, each containing 13.8 g or 6.9 g of powder. Each sachet contains the following ingredients: Macrogol (Polyethylene Glycol) 3350: 13.125g, Sodium Chloride: 350.7mg; Sodium Bicarbonate: 178.5mg; Potassium Chloride: 46.6mg; and flavouring and sweetener (trace amounts). The sachets include instructions for making the powder up in 125mL of water.

When temperature and humidity increase, the polyethylene glycol, especially polyethylene glycol 3350 structure is disrupted and an increase in impurities occurs. Carboxylic acid and formaldehyde are formed as impurity. Increasing impurity leads to a decrease in the amount of substances and excipients.

WO2005102364 (A1) discloses a compressed pharmaceutical composition comprising polyethylene glycol and sodium chloride, sodium bicarbonate, or potassium chloride. This invention includes an extra process for a compressed composition and the production method of the formulation is also not disclosed.

EP2459218 A1 discloses a formulation comprising polyethylene glycol having an average molecular weight of 2500 to 4500, sodium chloride, potassium chloride and sodium bicarbonates. In the application, a process is not disclosed and the formulation is in the form of a solution in water,

CN02772427 B discloses compositions comprising polyethylene glycol and electrolyte components sodium chloride, potassium choloride and sodium bicarbonate. The compositions are manufactured in a multi-step process, in which each component is independently pulverised, sieved, and are only brought together at the final mixing stage.

In the prior art, the compositions comprising polyethylene glycol preparation have the drawback that said compositions produces by extra process steps or unsuitable process for polyethylene glycol structure. In the process there is, however, the risk that polyethylene glycol might be decomposed through interactions with the solvent used or created temperature of extra process, that causes formation of undesirable degradation products.

In the present invention, easy process is used for the sachet formulation comprising polyethylene glycol. The process prevents the degradation of polyethylene glycol and it helps to develop a stable formulation.

### Detailed description of the Invention

The main object of the present invention is to provide a formulation with high stability which overcomes the above described problems in the prior art and have additive advantages over them.

The sachet formulation processed by dry mixing to protect the composition against the moisture and temperature to maintain the stability during process and shelf life.

The term "sachet" will refer to an envelope or bag for a granulate, while "granulate" refers to particles, granulate or spheronised particles. The sachets require no special shaping or molding operations. Thus, the compaction, compression or tamping steps used with other dosage forms are not needed.

Herein described are sachet formulations comprising polyethylene glycol with a molecular weight of 2500 to 4500 and at least one electrolyte wherein processed by an dry mixing process. So, the sachet formulation provides desired stability.

According to the present invention, polyethylene glycol with a molecular weight of 3350 is used in the method for manufacturing a sachet formulation.

AU 2015275229 A1 compositions for colon cleansing, wherein said compositions comprises polyethylene glycol; alkali metal sulphate, electrolytes selected from the group consisting of sodium bicarbonate, sodium chloride, and potassium chloride or a mixture thereof; and a gastro-protected dye composition. According to par. [0008] of AU 2015275229 A1; the composition may be in a sachet dosage form. Polyethylene glycol with a molecular weight between 3300 and 4000 g/mol is disclosed in AU 2015275229 A1. According to Ex-2 of AU 2015275229 A1, the sachet formulation contains 84.73% by weight of polyethylene glycol in the total formulation.

According to the present invention, the amount of polyethylene glycol ir the total formulation is between 92.0% and 98.0% by weight. These ratios are important in order to provide dissolution rate and effective treatment in the present formulation.

Polyethylene glycol is considered to be effective as a laxative by virtue of its osmotic action in the gut. Polyethylene glycol is not absorbed by the gut to any significant extent. When taken on its own, polyethylene glycol has an electrolyte leaching effect, and so it can lead to a drop in electrolyte levels in the patient. So, appropriate electrolytes are used in the present invention.

According to the present invention, the method concerns manufacturing a sachet formulation electrolytes. Sodium ions and potassium ions are particularly suitable. at least one electrolyte is selected from the group comprising sodium bicarbonate, sodium sorbate, sodium benzoate, sodium acetate, sodium carbonate, sodium citrate, sodium fumarate, sodium gluconate, sodium malate, sodium nitrate, sodium phosphate, sodium sulphate, potassium bicarbonate, potassium sorbate, potassium benzoate, potassium acetate, potassium carbonate, potassium citrate, potassium fumarate, potassium gluconate, potassium malate, potassium nitrate, potassium phosphate, potassium sulphate or mixtures thereof. Chloride and bicarbonate salts are particularly preferred. According to the invention, the method concerns manufacturing a sachet formulation comprising sodium chloride, potassium chloride and sodium bicarbonate.

According to one embodiment of the present invention, the amount of sodium chloride is between 1.5% and 3.5% by weight in the formulation.

According to one embodiment of the present invention, the amount of potassium chloride is between 0.01% and 1.0% by weight in the formulation.

According to one embodiment of the present invention, the amount of sodium bicarbonate is between 0.5% and 2.5% by weight in the formulation.

According to one embodiment of the present invention, all of using excipients are in the form of powder or granule or mixtures thereof.

According to one embodiment of the present invention, all of using excipients are in the form of powder.

According to one embodiment of the present invention, the method concerns manufacturing a sachet comprising:
85.0% - 98.0% by weight of polyethylene glycol
1.5% - 3.5% by weight of sodium chloride
0.01% - 1.0% by weight of potassium chloride
0.5% - 2.5% by weight of sodium bicarbonate of the total formulation.

### Example 1: The sachet formulation processed by dry mixing

| **Ingredients** | **(%) amount (w/w)** |
|---|---|
| Polyethylene glycol with a molecular weight of 2500 to 4500 | 92.0% - 98.0% |
| Sodium chloride | 2.0% - 3.0% |
| Potassium chloride | 0.1% - 1.0% |
| Sodium bicarbonate | 1.0% - 2.0% |
| **Total** | **100** |

### Example 2: The sachet formulation processed by dry mixing

| **Ingredients** | **(%) amount (w/w)** |
|---|---|
| Polyethylene glycol 3350 | 95.8 |
| Sodium chloride | 2.6 |
| Potassium chloride | 0.5 |
| Sodium bicarbonate | 1.3 |
| **Total** | **100** |

### Process for example 1 or 2;

The process for the preparation of the sachet formulation comprises the following steps:
a) Mixing polyethylene glycol, sodium chloride, potassium chloride and sodium bicarbonate,
b) Sieving the mixture and mixing again,
c) Filling the mixture into a sachet.

## Claims

1. Method for manufacturing a sachet formulation comprising polyethylene glycol and electrolyte wherein said formulation is produced by dry mixing process and wherein the amount of polyethylene glycol in the total formulation is between 92.0% and 98.0% by weight, comprising steps of
a) Mixing polyethylene glycol 3350, sodium chloride, potassium chloride and sodium bicarbonate,
b) Sieving the mixture and mixing again,
c) Filling the mixture into a sachet.

2. Method according to claim 1 wherein the amount of sodium chloride is between 1.5% and 3.5% by weight in the formulation.

3. Method according to claim 1 wherein the amount of potassium chloride is between 0.01% and 1.0% by weight in the formulation.

4. Method according to claim 1 wherein the amount of sodium bicarbonate is between 0.5% and 2.5% by weight in the formulation.

5. Method according to claim 1 wherein said excipients are in the form of powder or granule or mixtures thereof, preferably powder.

## Patentansprüche

1. Verfahren zur Herstellung einer Beutelformulierung, die Polyethylenglykol und Elektrolyt umfasst, wobei die Formulierung durch ein Trockenmischverfahren hergestellt wird und wobei die Menge an Polyethylenglykol in der Gesamtformulierung zwischen 92,0 Gew.-% und 98,0 Gew.-% liegt, umfassend die folgenden Schritte
a) Mischen von Polyethylenglykol 3350, Natriumchlorid, Kaliumchlorid und Natriumbicarbonat,
b) Sieben der Mischung und erneutes Mischen,
c) Einfüllen der Mischung in einen Beutel.

2. Verfahren nach Anspruch 1, wobei die Menge an Natriumchlorid zwischen 1,5 Gew.-% und 3,5 Gew.-% in der Formulierung liegt.

3. Verfahren nach Anspruch 1, wobei die Menge an Kaliumchlorid zwischen 0,01 und 1,0 Gewichtsprozent in der Formulierung liegt.

4. Verfahren nach Anspruch 1, wobei die Menge an Natriumbicarbonat zwischen 0,5 % und 2,5 Gew.-% in der Formulierung liegt.

5. Verfahren nach Anspruch 1, wobei die Hilfsstoffe in Form von Pulver oder Granulat oder Mischungen davon, vorzugsweise Pulver, vorliegen.

## Revendications

1. Procédé de fabrication d'une formulation en sachet comprenant du polyéthylène glycol et un électrolyte, dans lequel ladite formulation est produite par un procédé de mélange à sec et dans lequel la quantité de polyéthylène glycol dans la formulation totale est comprise entre 92,0 % et 98,0 % en poids, comprenant les étapes consistant à
a) Mélanger du polyéthylène glycol 3350, du chlorure de sodium, du chlorure de potassium et du bicarbonate de sodium,
b) Tamisage du mélange et nouveau mélange,
c) remplir le mélange dans un sachet.

2. Procédé selon la revendication 1, dans lequel la quantité de chlorure de sodium est comprise entre 1,5 % et 3,5 % en poids dans la formulation.

3. Procédé selon la revendication 1, dans lequel la quantité de chlorure de potassium est comprise entre 0,01 % et 1,0 % en poids dans la formulation.

4. Procédé selon la revendication 1, dans lequel la quantité de bicarbonate de sodium est comprise entre 0,5 % et 2,5 % en poids dans la formulation.

5. Procédé selon la revendication 1, dans lequel lesdits excipients se présentent sous forme de poudre ou de granulés ou de mélanges de ceux-ci, de préférence sous forme de poudre.
